(19) Europäisches Patentamt
European Patent Office
Office européen des brevets

(11) EP 2 039 754 B1

(12) EUROPEAN PATENT SPECIFICATION

(45) Date of publication and mention
of the grant of the patent:
**08.07.2020 Bulletin 2020/28**

(51) Int Cl.:
***C12M 1/02*** *(2006.01)*    ***C12M 1/06*** *(2006.01)*

(21) Application number: **08014470.2**

(22) Date of filing: **13.08.2008**

(54) **Bioreactor, cell culture method, and substance production method**

Bioreaktor, Zellkulturverfahren und Substanzherstellungsverfahren

Bioréacteur, procédé de culture cellulaire, et procédé de production de substance

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **21.09.2007 JP 2007244932**

(43) Date of publication of application:
**25.03.2009 Bulletin 2009/13**

(73) Proprietor: **Hitachi Plant Services Co., Ltd.**
**Tokyo 170-6034 (JP)**

(72) Inventors:
• **Amano, Ken**
**Tokyo 100-8220 (JP)**
• **Nakano, Ryusei**
**Tokyo 170-8466 (JP)**
• **Murakami, Sei**
**Tokyo 170-8466 (JP)**

(74) Representative: **Strehl Schübel-Hopf & Partner**
**Maximilianstrasse 54**
**80538 München (DE)**

(56) References cited:
**EP-A- 0 025 571        EP-A- 0 477 818**
**EP-A- 1 167 511        WO-A-94/12630**
**WO-A-03/057818      WO-A-2007/087438**
**US-A- 4 207 275       US-A- 4 454 078**
**US-A- 5 972 661       US-A- 5 972 695**
**US-A1- 2007 065 927**

**Description**

BACKGROUND OF THE INVENTION

Field of the Invention

The present invention relates to a bioreactor for culturing cells while providing aeration and agitation to a culturing tank, a cell culture method using the bioreactor, and a substance production method using the bioreactor.

Description of the Related Art

A culture tank for culturing microorganisms, such as Escherichia coli, has been widely used as a means for producing useful substances using microorganisms. In a culture tank for culturing microorganisms, a dissolved oxygen concentration is controlled so as to be maintained at a constant value by providing agitation to the tank using an impeller while aerating the culture tank with oxygen or air through a sparger. In the tank, oxygen gas in bubbles dissolves into a liquid and becomes dissolved oxygen, and microorganisms absorb the dissolved oxygen at a constant uptake rate. When this oxygen transfer rate from bubbles to the liquid and the oxygen uptake rate by the microorganisms equilibrate, the dissolved oxygen concentration in the culturing tank can be maintained to be constant. The oxygen uptake rate per unit volume of liquid can be obtained by multiplying an oxygen uptake rate per weight of microorganisms and the weight of microorganisms per unit volume of liquid, and is to be an approximately constant value through the entire region in the culture tank.

Meanwhile, while bubbles introduced from the sparger located on the bottom of the culture tank rises towards the liquid surface in an upper part of the culture tank, a partial pressure of oxygen in the bubbles decreases because the oxygen in the bubbles dissolves into the liquid. Accordingly, the oxygen transfer rate is lower in an upper part of the culture tank. Therefore, while the dissolved oxygen concentration in a lower part of the culture tank is high, the dissolved oxygen concentration on an upper part of the culture tank is low, resulting in generation of a concentration gradient in dissolved oxygen concentrations in the culture tank. This phenomenon is more significant with microorganisms having a larger oxygen uptake rate. The dissolved oxygen concentration in the culture tank is controlled on the basis of an average value of dissolved oxygen concentrations in the culture tank. However, both dissolved oxygen concentrations which are higher or lower than the average value are not preferable as a culture environment.

Spargers may be installed at multiple vertical positions in a culture tank in order to prevent a decrease in the dissolved oxygen concentration in an upper part of the tank. However, such a configuration has disadvantages that the structure becomes complicated due to arrangement of the spargers and that the control on the aeration volume becomes complicated.

US-A-5 972 661 discloses a mixer system with four impellers for alternately generating radial and axial flow of a non-Newtonian liquid mixed with gas in a container. US-A-5 972 695 discloses a fermenter with an upper helical and a lower turbine impeller.

SUMMARY OF THE INVENTION

Hence, the present invention has been conducted in view of the above-described actual situation, and an object of the present invention is to provide a bioreactor which is effective in uniforming the dissolved oxygen concentrations in vertical directions of a culture tank, a cell culture method using the bioreactor, and a substance production method using the bioreactor.

The present invention which has achieved the above-described object is defined in Claim 1. Further advantageous features are set out in the dependent claims.

In order to homogenize dissolved oxygen concentrations in vertical directions in the culture tank, it is necessary to equilibrate an oxygen uptake rate per unit volume of the culture tank with an oxygen transfer rate at any height position in the culture tank. The oxygen uptake rate per unit volume of the culture tank can be obtained by multiplying an oxygen uptake rate per biomass weight and the biomass weight per unit volume, and is to be an approximately constant value at any height position in the culture tank. Accordingly, the oxygen dissolving rate also needs to be a constant value. The oxygen dissolving rate is proportional to the product of a partial pressure of oxygen in bubbles and a mass transfer capacity coefficient $K_L a$. The partial pressure of oxygen in bubbles decreases as the bubbles rise from a lower part to an upper part of the culture tank. Since the bioreactor according to the present invention is configured to have a mass transfer capacity coefficient $K_L a$ increasing from a lower part to an upper part, a portion of a decrease in partial pressure of oxygen in bubbles can be compensated. As a result, it is possible to have a constant oxygen dissolving rate per unit volume.

It should be noted that "to homogenize dissolved oxygen concentrations in vertical directions in the culture tank" in the present invention does not mean that dissolved oxygen concentrations in any positions in a vertical direction of the culture tank show a completely identical value, but that the difference between a dissolved oxygen concentration in an upper part of the culture tank and a dissolved oxygen concentration in a lower part is smaller compared to that in a conventional bioreactor.

Here, a means for performing adjustment in the bioreactor according to the present invention such that $K_L a$ is to be large can be exemplified by increasing an outside diameter of an impeller, increasing the number of blades of an impeller, and increasing the rate of revolution of an impeller; however, it is not limited to these means. For example, a means for adopting a notched structure of an impeller in order to reinforce turbulence of a culture liquid by agitation using the impeller can be applied. Such means may be individually adopted and then an impeller is designed, or a combination of these means may be adopted and an impeller is designed.

In the meantime, according to the present invention, it is possible to provide a cell culture method, using the above-described bioreactor according to the present invention, in which cells are cultured while a medium filled in the culture tank is agitated by the multiple impellers. Furthermore, according to the present invention, it is also possible to provide a substance production method, using the above-described bioreactor according to the present invention, in which cells are cultured while a medium filled in the culture tank is agitated by the multiple impellers and a product produced by the cells is harvested from the medium.

With the bioreactor according to the present invention, it is possible to reduce the difference in dissolved oxygen concentration between an upper part and a lower part of the culture tank, and to homogenize dissolved oxygen concentrations in vertical directions of the culture tank.

Meanwhile, with the cell culture method according to the present invention, it is possible to culture cells at an excellent cell growth rate because the bioreactor achieving a cell culture environment in which dissolved oxygen concentrations in vertical directions of the culture tank are homogenized is used.

Moreover, with the substance producing method according to the present invention, it is possible to achieve excellent productivity because the bioreactor achieving a cell culture environment in which dissolved oxygen concentrations in vertical directions of the culture tank are homogenized is used.

BRIEF DESCRIPTION OF THE DRAWINGS

Fig. 1 is a schematic configuration diagram schematically illustrating an example of a bioreactor to which the present invention is applied.
Fig. 2 is a schematic configuration diagram illustrating an enlarged view of an impeller located in the bioreactor to which the present invention is applied.
Fig. 3 is a characteristics chart showing changes in physical quantity in a height direction of a culture tank.
Fig. 4 is a characteristics chart showing distribution of dissolved oxygen concentrations in a height direction of the culture tank.
Fig. 5 is a schematic configuration diagram schematically illustrating an example of a bioreactor to which the present invention is applied.
Fig. 6 is a schematic configuration diagram schematically illustrating an example of a bioreactor to which the present invention is applied.

Fig. 7 is a characteristics chart showing distributions of dissolved oxygen concentrations in height directions of the culture tank corresponding to respective Examples illustrated in Figs. 5 and 6.
Fig. 8 is a schematic configuration diagram schematically illustrating another example of a bioreactor to which the present invention is applied.

DESCRIPTION OF THE PREFERRED EMBODIMENTS

Detailed description will be given of the present invention below with reference to the drawings.

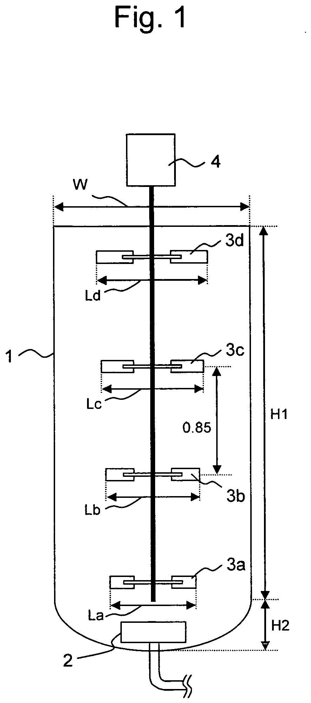

A bioreactor to which the present invention is applied includes, as shown in Fig. 1; a culture tank 1; a sparger 2 arranged at the bottom part of the culture tank 1; multiple impellers 3a, 3b, 3c, and 3d arranged in multiple stages in a vertical direction of the culture tank 1; and a control unit 4 having a motor and the like for rotary driving the impellers 3a, 3b, 3c, and 3d. Here, the bioreactor illustrated in Fig. 1 is configured to have the impellers 3a, 3b, 3c, and 3d respectively arranged in four stages; however, the technical scope of the present invention is not limited to such a configuration. To be more specific, a bioreactor according to the present invention needs to include impellers located in at least two stages. Accordingly, a configuration including impellers respectively arranged in 2 stages, a configuration including impellers respectively arranged in 3 stages, and a configuration including impellers respectively arranged in 4 stages, for example, may be employed.

Meanwhile a bioreactor according to the present invention may have any other configuration than the configurations illustrated in Fig. 1. For example, the present invention can be applied to a bioreactor including: multiple baffle plates arranged on the inner wall of the culture tank 1; a pump for introducing a gas through the sparger 2 into the culture tank 1; a medium feeder for feeding additional medium (feed medium) into the culture tank 1; a controller for controlling the amount of medium to be fed; a temperature measuring electrode for measuring the temperature of culture liquid; a pH sensor for measuring pH of the culture liquid; a DO sensor for measuring a dissolved oxygen concentration of the culture liquid; and the like.

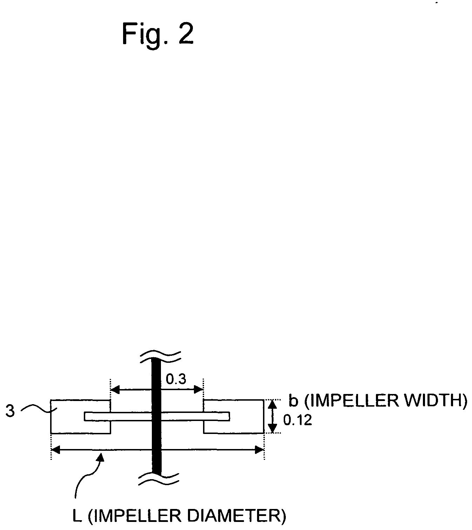

The impellers 3a, 3b, 3c, and 3d arranged in respective stages in the bioreactor illustrated in Fig. 1 each comprise 6 disk turbine blades. In the bioreactor shown in the present embodiment, the impellers 3a, 3b, 3c, and 3d (they are illustrated as impeller 3 in Fig. 2) arranged in respective stages each have a uniform impeller width and a uniform impeller inside diameter as shown in Fig. 2. However, the impeller outside diameters L are made sequentially larger from a lower stage such that the area of the respective impeller is sequentially increased. To be more specific, it is configured so that the impeller outside diameter L of the impeller 3a is the smallest, followed by those of the impeller 3b, the impeller 3c, and the impeller 3d. Here, the impellers 3a, 3b, 3c, and 3d are rotary driven on the same axis by the controller 4. Accordingly, they have the same number of revolutions. Therefore, it is configured so that an impeller located in an upper stage always has larger agitation power and a larger mass transfer capacity coefficient $K_La$ than an impeller located in a lower stage. Note that, the bioreactor illustrated in Fig. 1 is configured such that the mass transfer capacity coefficient $K_La$ of an impeller located in an upper stage is larger than that of an impeller located in a lower stage by making the impeller outside diameters L of the impellers sequentially larger from a lower stage; however, the technical scope of the present invention is not limited to such a configuration.

For example, total areas of the impellers located in respective multiple stages can be designed to become sequentially larger from a lower stage by making the impeller width of the impellers 3a, 3b, 3c, and 3d sequentially larger from a lower stage. Alternatively, total areas of the impellers located in respective multiple stages can be designed to become sequentially larger from a lower stage by making the number of blades of the impellers located in respective multiple stages sequentially larger from a lower stage. In other words, the shape or the number of blades of individual impellers located in respective stages can be appropriately designed so that total areas of impellers located in respective multiple stages can be made sequentially larger from a lower stage.

Meanwhile, the bioreactor according to the present invention may include multiple controllers so as to rotary drive the impellers 3a, 3b, 3c, and 3d independently. In this case, the mass transfer capacity coefficient $K_La$ of an impeller located in an upper stage can be larger than the mass transfer capacity coefficient $K_La$ of an impeller located in a lower stage by controlling such that the numbers of revolutions of the impellers 3a, 3b, 3c, and 3d can be sequentially larger from a lower stage.

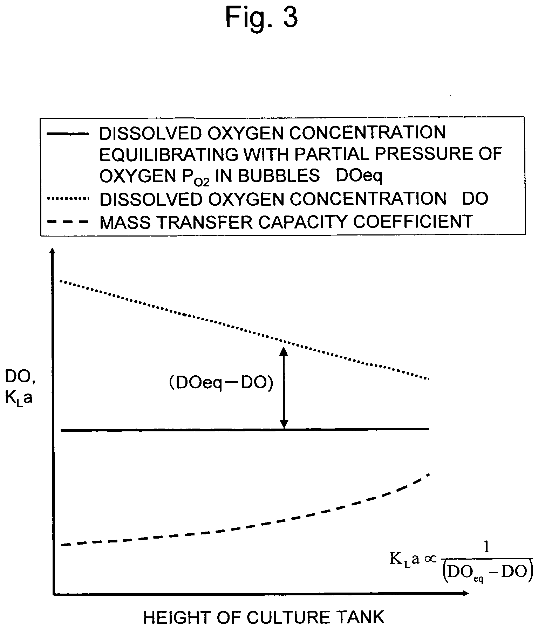

The theory regarding the distribution of dissolved oxygen concentrations in the culture tank 1 in the bioreactor according to the present invention is shown in Fig. 3. Here, the amount of oxygen dissolving into the liquid per unit volume per unit time is:

$$K_La\left(DO_{eq} - DO\right) \quad (\mathrm{mg/L/s}) \quad \cdots\cdots (1)$$

Here, $K_La$ represents a mass transfer capacity coefficient (1/s). DO represents a dissolved oxygen concentration (mg/L) in the liquid, and is assumed to be constant in the culture tank. $DO_{eq}$ represents a dissolved oxygen concentration (mg/L) equilibrating with a partial pressure of oxygen $P_{O2}$ in bubbles. According to the Henry's law, $DO_{eq}$ is proportional to a partial pressure of oxygen $P_{O2}$. An oxygen uptake rate (OUR) (mg/L/s) per unit volume by biomass is assumed to be constant in the culture tank. In order to achieve a constant dissolved oxygen concentration DO in the culture tank, ideally,

$$K_La\left(DO_{eq} - DO\right) = OUR \quad \cdots\cdots (2)$$

is satisfied throughout the culture tank. Suppose that this condition is satisfied, oxygen in bubbles decreases at a certain rate in the height direction since the oxygen uptake rate OUR is constant. Accordingly, the partial pressure of oxygen $P_{O2}$ in bubbles linearly decreases as shown in Fig. 3. Therefore, the dissolved oxygen concentration $DO_{eq}$ which equilibrates with the partial pressure of oxygen $P_{O2}$ in bubbles also linearly decreases.

In order to achieve a constant value of the oxygen dissolving rate formula (1), $K_La$ needs to be increased in the height direction so as to compensate a decrease of ($DO_{eq}$ - DO). As shown in Fig. 3, the impeller size needs to be determined according to

$$K_La \propto \frac{1}{\left(DO_{eq} - DO\right)} \quad \cdots\cdots (3)$$

such that $K_La$ increases in the height direction. It should be note that, in the case of using a disk turbine blade as an impeller, there is a design equation for estimating the agitation power and $K_La$ of the disk turbine blade. Accordingly, the impeller size can be designed by using the design equation such that the $K_La$ increases. Alternatively, for other general impeller shapes, an impeller size providing a desired $K_La$ can be determined by using numerical simulation of turbulence (R. Djebbar, M. Roustan, and A. Lane, "Numerical Computation of Turbulent Gas-Liquid Dispersion in mechanically Agitated Vessels," Transactions of Institution of Chemical Engineers, Vol. 74 part 1 (1996) pp. 492-498).

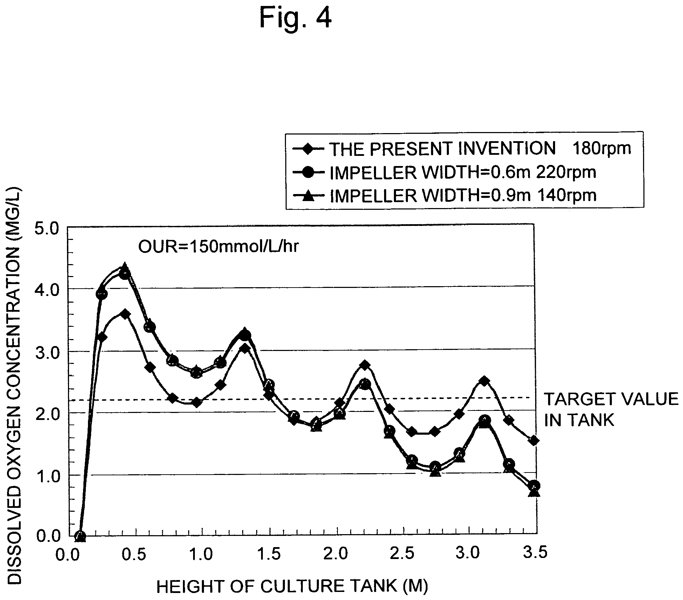

Results obtained by setting actual numerical values to various sizes in the bioreactor illustrated in Fig. 1 and then calculating dissolved oxygen concentrations at respective height positions in the culture tank are shown in Fig. 4. Here, various sizes of the bioreactor were set as follows. Firstly, the height HI of the culture tank 1, the height H2 of the bottom part of the culture tank 1, and the width W of the culture tank 1 were set to 3.0 (m), 0.45 (m), and 1.8 (m), respectively. Then, the impeller outside diameter La of the impeller 3a, the impeller outside diameter Lb of the impeller 3b, the impeller outside diameter Lc of the impeller 3c, and the impeller outside diameter Ld of the impeller 3d were set to 0.64 (m), 0.67 (m), 0.72 (m), and 0.79 (m), respectively. Meanwhile, the impellers 3a, 3b, 3c, and 3d were respectively set to have an impeller inside diameter A of 0.3 (m) and an impeller width B of 0.12 (m). Then, the number of revolutions of the impellers was set to 180 rpm.

Dissolved oxygen concentrations at respective height positions in the culture tank were calculated based on the above-described actual settings, and exhibited a profile illustrated by a curve connecting squares in Fig. 4. Here, for comparison, the same calculation was carried out for a configuration in which the impellers 3a, 3b, 3d, and 3d were all set to have an impeller outside diameter of 0.6 m and the number of revolutions was set to 220 rpm (a curve connecting circles), and for a configuration in which all of the impellers were set to have an impeller outside diameter of 0.9 m and the number of revolutions was set to 140 rpm (a curve connecting triangles). Here, the various designs and calculations were based on analysis using numerical simulation.

Numbers of agitations in these three analysis cases were different from each other so that the average value of agitation power in the tank could be the same among these cases. Agitation power is generally larger when an impeller outside diameter is larger. Accordingly, the number of agitations is to be reduced for a culture tank having a large impeller outside diameter. Therefore, the number of revolutions for the culture tank having an impeller outside diameter of 0.6 m was set to 220 rpm, while that for the culture tank having an impeller outside diameter of 0.9 m was set to 140 rpm. Since the impeller outside diameter of each of the impellers located in respective stages in the present Example was larger than 0.6 m and smaller than 0.9 m, the number of revolutions was set to an intermediate value of 180 rpm. The oxygen uptake rate of biomass was set to 150 mmol/L/hr, and the average dissolved oxygen concentration in the tank was designed to be 2.2 mg/L.

According to the results shown in Fig. 4, it is observed, in the culture tank in which all of the impellers located in respective 4 stages have the same impeller outside diameter, that the dissolved oxygen concentration exceeds 4 mg/L in a lower part of the culture tank while it fell below 1 mg/L in an upper part of the culture tank, resulting in generation of a large concentration gradient in the vertical direction. On the other hand, it is observed, in the culture tank of the present Example, that the dissolved oxygen concentration was approximately 3.5 mg/L in a lower part of the culture tank while it was approximately 1.5 mg/L in an upper part of the culture tank; therefore, the concentrations were homogenized in the vertical direction. It should be noted that, since the present Example has a distribution in which $K_L a$ is largest at positions where the respective impellers are attached and is reduced in the surrounding parts, distribution of the dissolved oxygen concentrations in the height direction has 4 peaks, and does not become the ideal pattern shown in Fig. 3.

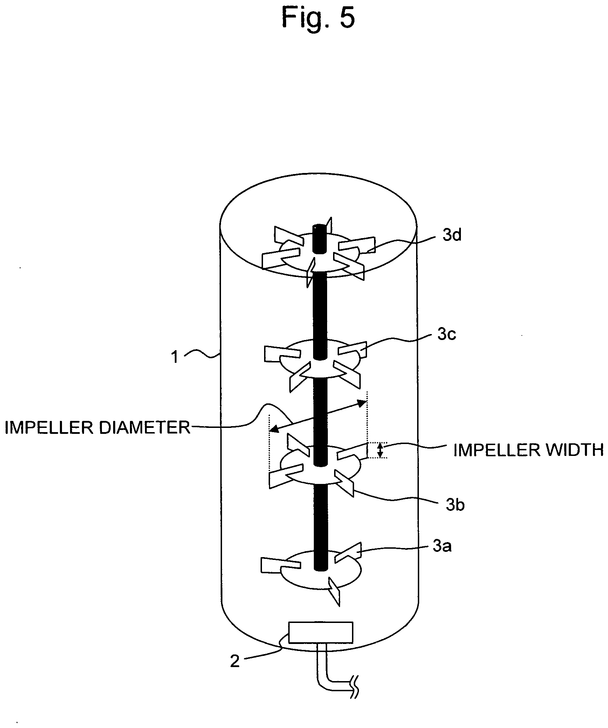

As another embodiment of a bioreactor according to the present invention, multiple impellers 3a, 3b, 3c, and 3d may be configured, as shown in Fig. 5, such that the numbers of blades increase from a lower stage to an upper stage. For example, the numbers of blades of the impellers 3a, 3b, 3c, and 3d can be set to 3, 4, 5, and 6, respectively. Note that, in this case, all of the impellers 3a, 3b, 3c, and 3d can be configured to have the same shape (for example, a shape having an impeller diameter of 0.9 m and an impeller width of 0.12 m). By setting the impellers 3a, 3b, 3c, and 3d as shown in Fig. 5, the mass transfer capacity coefficient $K_L a$ can be larger in an upper stage than in a lower stage.

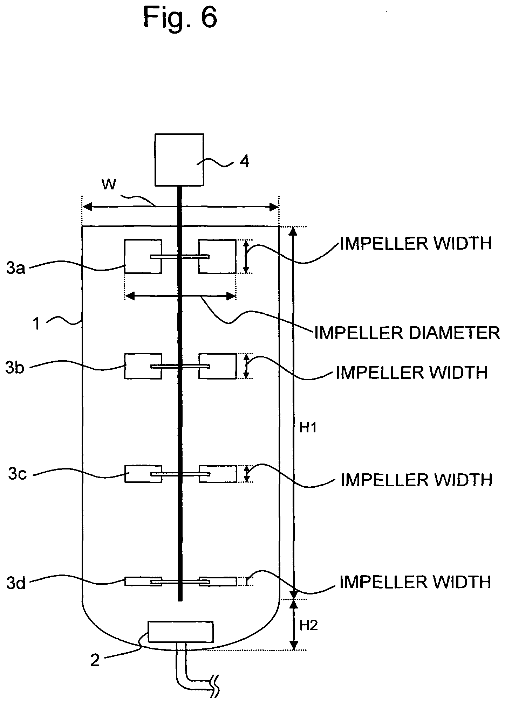

Moreover, as another embodiment of a bioreactor according to the present invention, multiple impellers 3a, 3b, 3c, and 3d may be configured, as shown in Fig. 6, such that the impeller widths increase from a lower stage to an upper stage. For example, the impeller widths of the impellers 3a, 3b, 3c, and 3d can be set to 0.06 m, 0.12 m, 0.18 m, and 0.24 m, respectively (the impeller diameter is fixed to 0.9 m). Note that, in this case, all of the impellers 3a, 3b, 3c, and 3d can be configured to have the same number of blades. By setting the impellers 3a, 3b, 3c, and 3d as shown in Fig. 6, the mass transfer capacity coefficient $K_L a$ can be larger in an upper stage than in a lower stage.

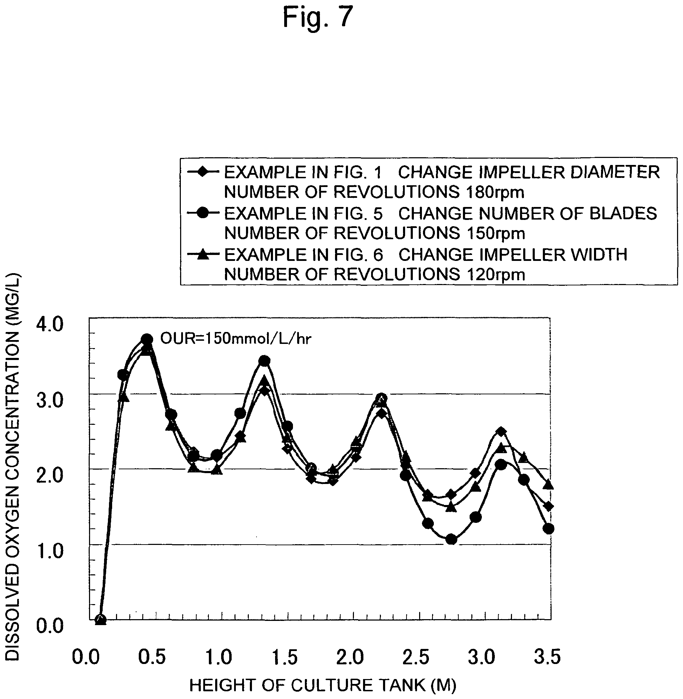

Here, in the bioreactors illustrated in Fig. 5 and Fig. 6, the sizes other than those of the impellers 3a, 3b, 3c, and 3d can be set to the same as those of the bioreactor illustrated in Fig. 1. In the bioreactors illustrated in Fig. 5 and Fig. 6, total impeller areas of the respective impellers 3a, 3b, 3c, and 3d are configured to increase sequentially from a lower stage to an upper stage. Results of the analysis of dissolved oxygen concentrations in the vertical direction of the culture tank in the bioreactors illustrated in Fig. 5 and Fig. 6 are shown in Fig. 7. In Fig. 7, a curve connecting squares represents the result of the analysis on the bioreactor illustrated in Fig. 1, a curve connecting circles represents the result of the analysis on the bioreactor illustrated in Fig. 5, and a curve connecting triangles represents the result of the analysis on the bioreactor illustrated in Fig. 6. The number of agitations in each of the bioreactors was adjusted so that the agitation power can be the same.

It is found in Fig. 7 that it is not be easy to provide an optimal design due to a small degree of freedom in design in the case of varying the number of blades as in the bioreactor illustrated in Fig. 5. On the other hand, it is observed in Fig. 7 that it is possible to homogenize dissolved oxygen concentrations similarly to the way in the bioreactor illustrated in Fig. 1 in the case, as in the bioreactor illustrated in Fig. 6, where impeller widths are varied such that impeller areas increase from a lower stage to an upper stage.

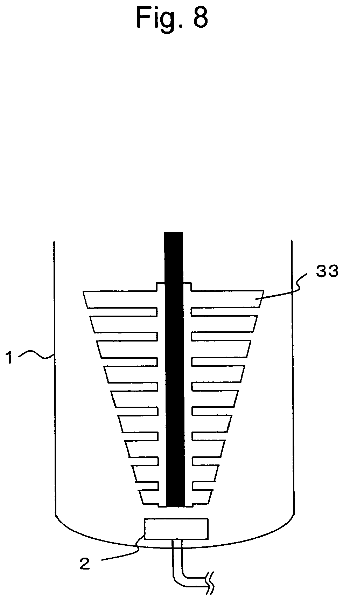

In the meantime, as another embodiment of a bioreactor according to the present invention, an impeller 33 having comb-like notches as shown in Fig. 8 can be exemplified. The impeller 33 has a shape in which the width becomes larger towards the upper part of the culture tank 1 from the bottom part thereof, and provided with multiple notched portions formed in parallel from the bottom of the culture tank 1 towards the upper part thereof. With the impeller 33 illustrated in Fig. 8, it is possible to achieve an ideal distribution, as shown in Fig. 3, in which dissolved oxygen concentrations are constant in the height direction. The impeller 33 illustrated in Fig. 8 has the impeller outside diameter continuously upsizing towards the upper part of the culture tank 1; therefore, a larger agitation power and $K_L a$ can be obtained as the position goes up. In addition, having comb-like notches, the impeller 33 can obtain high flow shear stress at the multiple notched portions; therefore, it is effective to obtain a high $K_L a$.

In a bioreactor according to the present invention, the sparger 2 is not particularly limited as long as it is configured to be capable of supplying a gas containing oxygen to a culture liquid inside of the culture tank 1. For example, a circular pipe provided with multiple holes for aeration formed on the surface thereof may be cited. As for the sparger 2, a cylindrical member made of a porous material can be exemplified. Here, as the porous material, a metal sintered body, an organic polymer porous material, a tetrafluoroethylene resin, stainless, sponge, and pumice stone, for example, can be employed. By having such a configuration, a gas supplied from a pump means, which is not shown in the drawing, can be supplied through the sparger 2 to a medium inside of the culture tank.

With a bioreactor according to the present invention configured as described above, it is possible to culture desired cells in a medium filled in the culture tank while providing aeration and agitation to the medium. Here, as for cells to be cultured, there is no limitation at all; however, examples are cells for producing substances which can be used as main raw materials of pharmaceutical products and the like. Moreover, there is no limitation to cells to be cultured, and examples include animal cells, plant cells, insect cells, bacteria, yeast, fungi, and algae. Especially, the cell culture method according to the present invention is preferably applied to culturing animal cells producing proteins, such as antibodies and enzymes. In the present invention, a substance to be produced is not limited in any way, and examples are proteins, such as antibodies and enzymes, and physiologically active substances, such as low-molecular-weight compounds and high-molecular-weight compounds.

Especially, in the bioreactor according to the present invention, a medium can be agitated so that dissolved oxygen concentrations are uniform in the height direction of the culture tank 1. Accordingly, cells can be cultured under uniform culture conditions in the height directions of the culture tank 1 in the bioreactor; therefore, it is possible to improve growth of cells to be cultured or productivity of target products from the cells. In addition, with the bioreactor according to the present invention, there is an effect of being able to keep an operation width to be small on the amount of aeration provided to the culture tank 1 and on the number of revolutions of the impeller 3. For example, in the case of fed batch culture, the amount of liquid and the oxygen uptake rate increase as the culture progresses. For this reason, in a culture tank having the same impeller outside diameter in all the stages, it is necessary to increase the number of revolutions and the amount of aeration as the culture progresses. On the other hand, in the culture tank of the present invention, $K_L a$ in the tank naturally increases as the amount of liquid increases without changing the number of revolutions and the amount of aeration. Accordingly, the operation width on the amount of aeration and the number of revolutions during the operation can be kept small. Especially, according to the configuration illustrated in Fig. 8, in the case where $K_L a$ continuously changes in accordance with the liquid level, it is also possible in principle to operate from the beginning to the end of the culture at a constant amount of aeration and a constant number of revolutions.

Explanation of Reference Numerals

1 ... culture tank, 2 ... sparger, 3 ... impeller

**Claims**

1. A bioreactor, comprising:

   a culture tank (1);
   sparger means (2) arranged in a lower part of the culture tank;
   a plurality of impellers (3a to 3d) being arranged in multiple stages in a vertical direction of the culture tank; and
   a controller (4) for individually controlling rotational driving of the plurality of impellers;
   wherein the plurality of impellers have a larger mass transfer capacity coefficient $K_L a$ per unit number of revolutions in an impeller located at an upper stage than in an impeller located at a lower stage.

2. The bioreactor according to claim 1, wherein the plurality of impellers (3a to 3d) have a larger impeller outside diameter (L) in an upper stage than in a lower stage.

3. The bioreactor according to claim 1, wherein the plurality of impellers (3a to 3d) have a larger number of blades in an upper stage than in a lower stage.

4. The bioreactor according to claim 1, wherein the culture tank (1) has such a height that a difference in dissolved oxygen concentration in the vertical direction is at least 3.0 mg/L.

5. A cell culture method comprising culturing cells by using the bioreactor according to any one of claims 1 to 4, while agitating a medium filled in the culture tank by use of the plurality of impellers.

6. A substance producing method, comprising:

   culturing cells by using the bioreactor according to any one of claims 1 to 4, while agitating a medium filled in the culture tank by use of the plurality of impellers; and

harvesting a product produced by the cells from the medium.

**Patentansprüche**

1. Bioreaktor, umfassend:

   einen Kulturtank (1);
   eine in einem unteren Teil des Kulturtanks angeordnete Sprinklereinrichtung (2);
   mehrere Impeller (3a-3d), die in mehreren Stufen in einer senkrechten Richtung des Kulturtanks angeordnet sind; und
   eine Steuerung (4) zur individuellen Steuerung des Rotationsantriebs der mehreren Impeller;
   wobei die mehreren Impeller einen größeren Massentransferkapazitätskoeffizienten $K_La$ pro Einheitsanzahl von Umdrehungen in einem an einer oberen Stufe befindlichen Impeller als in einem an einer unteren Stufe befindlichen Impeller aufweisen.

2. Bioreaktor nach Anspruch 1, wobei die mehreren Impeller (3a-3d) einen größeren Impelleraußendurchmesser L in einer oberen Stufe als in einer unteren Stufe aufweisen.

3. Bioreaktor nach Anspruch 1, wobei die mehreren Impeller (3a-3d) eine größere Anzahl von Schaufeln in einer oberen Stufe als in einer unteren Stufe aufweisen.

4. Bioreaktor nach Anspruch 1, wobei der Kulturtank (1) eine solche Höhe aufweist, dass eine Differenz in der Konzentration von gelöstem Sauerstoff in der senkrechten Richtung mindestens 3,0 mg/L ist.

5. Zellkulturverfahren, umfassend das Kultivieren von Zellen unter Verwendung des Bioreaktors nach einem der Ansprüche 1 bis 4, während ein in den Kulturtank eingeführtes Medium unter Verwendung der mehreren Impeller gerührt wird.

6. Substanzerzeugungsverfahren, umfassend:

   Kultivieren von Zellen unter Verwendung des Bioreaktors nach einem der Ansprüche 1 bis 4, während ein in den Kulturtank eingefülltes Medium unter Verwendung der mehreren Impeller gerührt wird; und
   Gewinnen eines von den Zellen erzeugten Erzeugnisses aus dem Medium.

**Revendications**

1. Bioréacteur, comprenant :

   un réservoir de culture (1) ;
   un moyen aérateur (2) disposé dans une partie inférieure du réservoir de culture ;
   une pluralité de roues à ailettes (3a à 3d) étant disposées en plusieurs étages dans une direction verticale du réservoir de culture ; et
   un dispositif de commande (4) pour commander individuellement l'entraînement en rotation de la pluralité de roues à ailettes ;
   dans lequel la pluralité de roues à ailettes ont un coefficient de capacité de transfert de masse $K_La$ plus élevé par unité de tours dans une roue à ailettes située à un étage supérieur que dans une roue à ailettes située à un étage inférieur.

2. Bioréacteur selon la revendication 1, dans lequel la pluralité de roues à ailettes (3a à 3d) ont un diamètre extérieur de roue à ailettes (L) plus grand dans un étage supérieur que dans un étage inférieur.

3. Bioréacteur selon la revendication 1, dans lequel la pluralité de roues à ailettes (3a à 3d) ont un plus grand nombre de pales dans un étage supérieur que dans un étage inférieur.

4. Bioréacteur selon la revendication 1, dans lequel le réservoir de culture (1) a une hauteur telle qu'une différence de concentration en oxygène dissous dans la direction verticale est d'au moins 3,0 mg/l.

5. Procédé de culture cellulaire comprenant la culture de cellules en utilisant le bioréacteur selon l'une quelconque des revendications 1 à 4, tout en agitant un milieu rempli dans le réservoir de culture par l'utilisation de la pluralité de roues à ailettes.

6. Procédé de production d'une substance, comprenant :

   la culture de cellules en utilisant le bioréacteur selon l'une quelconque des revendications 1 à 4, tout en agitant un milieu rempli dans le réservoir de culture par l'utilisation de la pluralité de roues à ailettes ; et
   la récolte d'un produit, produit par les cellules, à partir du milieu.

# Fig. 1

# Fig. 2

L (IMPELLER DIAMETER)

b (IMPELLER WIDTH)

# Fig. 3

—— DISSOLVED OXYGEN CONCENTRATION
EQUILIBRATING WITH PARTIAL PRESSURE OF
OXYGEN $P_{O2}$ IN BUBBLES   DOeq
············ DISSOLVED OXYGEN CONCENTRATION   DO
‒ ‒ ‒ MASS TRANSFER CAPACITY COEFFICIENT

DO,
$K_L a$

(DOeq−DO)

$$K_L a \propto \frac{1}{\left(DO_{eq} - DO\right)}$$

HEIGHT OF CULTURE TANK

# Fig. 4

# Fig. 5

IMPELLER DIAMETER

IMPELLER WIDTH

3d

3c

3b

3a

1

2

# Fig. 6

# Fig. 7

**Legend:**
- EXAMPLE IN FIG. 1 CHANGE IMPELLER DIAMETER NUMBER OF REVOLUTIONS 180rpm
- EXAMPLE IN FIG. 5 CHANGE NUMBER OF BLADES NUMBER OF REVOLUTIONS 150rpm
- EXAMPLE IN FIG. 6 CHANGE IMPELLER WIDTH NUMBER OF REVOLUTIONS 120rpm

OUR=150mmol/L/hr

Y-axis: DISSOLVED OXYGEN CONCENTRATION (MG/L)
X-axis: HEIGHT OF CULTURE TANK (M)

Fig. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5972661 A

- US 5972695 A

**Non-patent literature cited in the description**

- **R. DJEBBAR ; M. ROUSTAN ; A. LANE.** Numerical Computation of Turbulent Gas-Liquid Dispersion in mechanically Agitated Vessels. *Transactions of Institution of Chemical Engineers,* 1996, vol. 74, 492-498